(19) Europäisches Patentamt
European Patent Office
Office européen des brevets

(11) Veröffentlichungsnummer: **0 054 699**
**B1**

(12) **EUROPÄISCHE PATENTSCHRIFT**

(45) Veröffentlichungstag der Patentschrift: **14.03.84**

(51) Int. Cl.³: **C 07 C 41/28**, C 07 C 43/205

(21) Anmeldenummer: **81109004.2**

(22) Anmeldetag: **27.10.81**

(54) **Verfahren zur katalytischen Hydrogenolyse von p-substituierten Benzaldehyd-dimethylacetalen zu den entsprechenden Benzylmethyl-ether-Derivaten.**

(30) Priorität: **24.12.80 DE 3048993**

(43) Veröffentlichungstag der Anmeldung: **30.06.82 Patentblatt 82/26**

(45) Bekanntmachung des Hinweises auf die Patenterteilung: **14.03.84 Patentblatt 84/11**

(84) Benannte Vertragsstaaten: **AT BE CH DE FR GB IT LI NL SE**

(56) Entgegenhaltungen:
**US - A - 4 201 868**

**CHEMICAL ABSTRACTS, Band 85, Nr. 7, 16. August 1976, Seite 453, Nr. 45686z, Columbus, Ohio, USA, B.I. FLEMING et al.: "The reduction of acetals with cobalt carbonyl catalysts"**

(73) Patentinhaber: **HOECHST AKTIENGESELLSCHAFT, Postfach 80 03 20, D-6230 Frankfurt am Main 80 (DE)**

(72) Erfinder: **Fischer, Hartmut, Dr., Breckenheimer Strasse 32, D-6238 Hofheim (DE)**
Erfinder: **Skaletz, Detlef, Dr., Südring 281, D-6500 Mainz (DE)**

**EP 0 054 699 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur katalytischen Hydrogenolyse von p-substituierten Benzaldehyd-dimethylacetalen zu den entsprechenden Benzylmethyl-ether-Derivaten

Die Erfindung betrifft ein Verfahren zur katalytischen Hydrogenolyse von p-substituierten Benzaldehyd-dialkylacetalen zu den entsprechenden p-substituierten Benzylalkylether-Derivaten.

Die Hydrogenolyse von Benzaldehyd-dialkylacetalen ist bekannt und führt unter Eliminierung beider Alkoxygruppen zu Toluol. Diese Reaktion tritt nahezu quantitativ ein, wenn die Hydrogenolyse mit Palladium- oder Platinkatalysatoren durchgeführt wird; man verwendet diese Reaktion beispielsweise, um einen als Schutzgruppe in ein Molekül eingeführten Benzylrest selektiv wieder aus dem Molekül zu entfernen (siehe dazu beispielsweise P. N. Rylander, Catalytic Hydrogenation over Platinum Metals, Academic Press – New York and London, 1967, Seiten 449 bis 454).

Zur Abspaltung eines Methoxyrestes aus dem Dimethylacetal des Benzaldehyds, um den entsprechenden Benzylmethylether zu synthetisieren, sind die Katalysatoren der Benzylspaltung jedoch völlig ungeeignet. Es ist aber aus dem Stand der Technik bekannt, Benzaldehyd-dialkylacetale dadurch zu Benzylalkylethern zu hydrogenolysieren, daß man als Katalysator Kobaltcarbonyl verwendet und die Acetale mit einem Gemisch aus Kohlenmonoxid und Wasserstoff umsetzt: B. I. Fleming und H. I. Bolker beschreiben in einem Aufsatz »The reduction of acetals with cobalt carbonyl catalysts« (Die Reduktion von Acetalen mit Kobaltcarbonyl-Katalysatoren) in Can. J. Chem., Vol. 54, 1976, Seiten 685 bis 694, die Umsetzung von Benzaldehyd-dimethylacetal (IIa), Benzaldehyd-dibenzylacetal (IIb) und den p-substituierten Verbindungen 4-Methoxy-benzaldehyd-dibenzylacetal (IIc) und 4-Chlor-benzaldehyd-dibenzylacetal (IId)

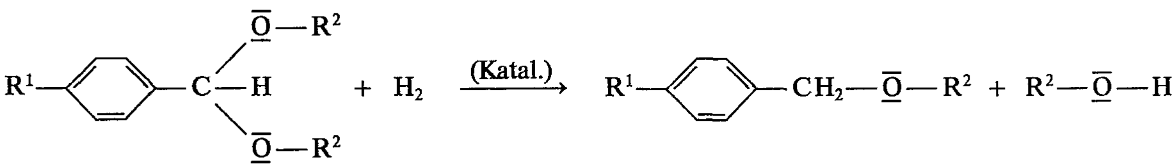


(IIa–d) (IIIa–d)

| Edukt | $R^1$ | $R^2$ | Produkt |
|---|---|---|---|
| IIa | H | $CH_3$ | IIIa |
| b | H | $CH_2$— 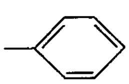 | b |
| c | $\overline{O}-CH_3$ | $CH_2$— 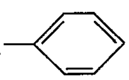 | c |
| d | Cl | $CH_2$— 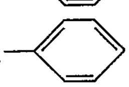 | d |

in 96%iger Ausbeute zum Benzylmethylether (IIIa), in 95% zum Dibenzylether (IIIb), in 59% zum (4-Methoxy-benzyl)-benzylether (IIIc) und in 16% zum (4-Chlor-benzyl)-benzylether (IIId). Als Katalysator wird $Co_2(CO)_8$ in Benzol eingesetzt und das Verhältnis von $H_2$ zu CO beträgt 2 zu 1.

Wenn man dieses bekannte Verfahren auf solche Dimethylacetale von Benzaldehyden überträgt, die in p-Stellung eine Alkoxy-, Aryloxy- oder Aralkoxygruppe aufweisen, d. h. beispielsweise auf 4-Methoxy-benzaldehyd-dimethylacetal oder auf 4-Phenoxy-benzaldehyd-dimethylacetal, so verläuft diese katalytische Hydrogenolyse nur wenig selektiv. So erhält man bei der Hydrogenolyse des 4-Methoxy-benzaldehyd-dimethylacetals (IV) mit Kobaltcarbonyl als Katalysator den 4-Methoxybenzyl-methylether (Va) nur zu etwa 10 bis 12%. Als weitere Produkte werden in Ausbeuten von etwa 49 bzw. 40% 4-Methoxy-toluol (Vb) und (4-Methoxy-phenyl)-acetaldehyd-dimethylacetal (Vc) gebildet:

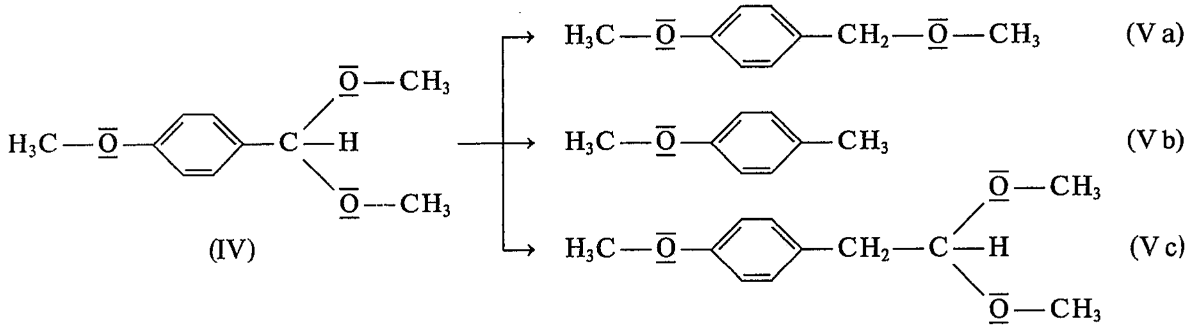

Man kann wohl annehmen, daß die Verbindung (Vb) durch »Benzylether«-Spaltung aus (IV) entsteht und die Verbindung (Vc) durch Einschub von Kohlenmonoxid in die Acetalgruppe, wobei durch daran anschließende Hydrierung die Kette um eine $CH_2$-Gruppe verlängert wird.

Sehr ähnlich verhält sich das Bis-dimethylacetal des 4,4'-Diphenylether-dialdehyds (VI), das erstmals in der gleichzeitig eingereichten Patentanmeldung EP-A-0 054 698 (interne Bezeichnung Hoe 80/K 092) mit dem Titel »4,4'-Diphenylether-dialdehyd-bis-dimethylacetal und ein Verfahren zu seiner Herstellung« beschrieben wird. Es ergeben sich bei der Hydrogenolyse in Anlehnung an B. I. Fleming et al. (s. o.) 26% des 4,4'-Bis(methoxymethyl)-diphenylethers (VII), während die Benzyletherspaltung zu 16% und die Kohlenmonoxideinschiebung zu 20% ablaufen; daneben entstehen noch mehrere unbekannte Produkte, so daß die Hydrogenolyse von (VI) unter bekannten Verfahrensbedingungen ausgesprochen unselektiv verläuft.

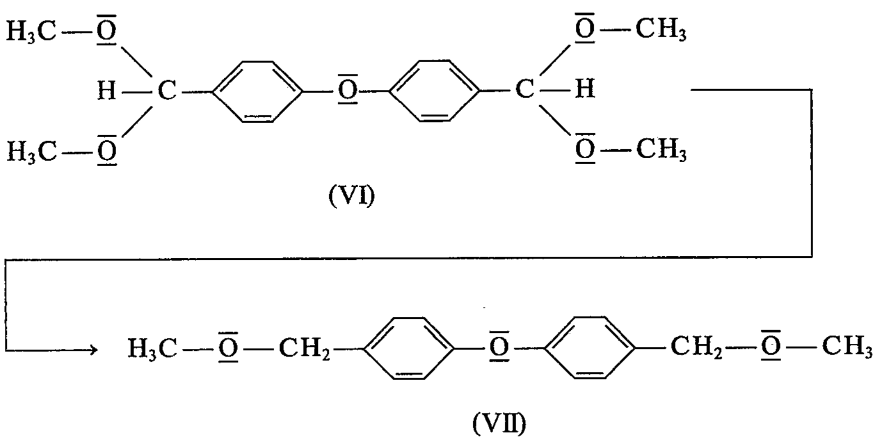

+ diverse Produkte

Es sind in der Literatur auch bereits Modifikationen von Kobaltcarbonyl-Katalysatoren beschrieben worden, die zu unterschiedlichsten Ergebnissen führen:

Beispielsweise wird in »Organic Syntheses via Metal Carbonyls« (Organische Synthesen über Metallcarbonyle), herausgegeben von I. Wender und P. Pino, Interscience Publishers — New York, 1968, Seiten 84 bis 86, ausgeführt, daß Basen bei der Einwirkung auf Kobaltcarbonyl dessen Disproportionierung zu ionischen Komplexen einleiten, was auch als »Basenreaktion« bezeichnet wird; dadurch kann das Kobaltcarbonyl als katalytisch aktive Verbindung eines homogenen Reaktionssystems in unwirksame Ionenpaare überführt werden.

In den Aufsätzen »Novel Hydroformylation Catalysts« (Neue Hydroformylierungs-Katalysatoren) von L. H. Slaugh und R. D. Mullineaux in J. Organometal. Chem., Vol. 13, 1968, Seiten 469 bis 477, und »Homogeneous Hydrogenation of Ketones Catalyzed by Cobalt Carbonyl Phosphine Complexes« (Durch Kobaltcarbonyl-Phosphin-Komplexe katalysierte homogene Hydrierung von Ketonen) von L. Marko, B. Beil und S. Vastag in Homogeneous Catalysis—II, Advances in Chemistry Series 132 der American Chemical Society, 1974, Seiten 27 bis 32, wird vorgeschlagen, zur Steuerung der Selektivität von Kobaltcarbonyl-Katalysatoren diese mit bestimmten organischen Phosphorverbindungen zu modifizieren; diese bekannte Methode versagt jedoch bei Einsatz in der Hydrogenolyse der einleitend angegebenen Acetale. Mit Kobaltcarbonylen, die Triphenylphosphan, Tri-n-butylphosphan oder Triethylphosphit enthielten, konnte keine Hydrogenolyse dieser Acetale erreicht werden.

Der Erfindung liegt deshalb die Aufgabe zugrunde, ein Katalysatorsystem zu finden, das es ermöglicht, auch bestimmte p-substituierte Benzaldehyd-dimethylacetale mit verhältnismäßig hoher Selektivität in die entsprechenden Benzylmethylether zu überführen.

Die Erfindung geht aus von dem bekannten Verfahren zur katalytischen Hydrogenolyse von p-substituierten Benzaldehyd-dialkylacetalen mit einem Kobaltcarbonyl-Katalysator und einem Gemisch aus Wasserstoff/Kohlenmonoxid zu den entsprechenden p-substituierten Benzylalkylether-Derivaten. Das erfindungsgemäße Verfahren ist dann dadurch gekennzeichnet, daß man Verbindungen der allgemeinen Formel (I)

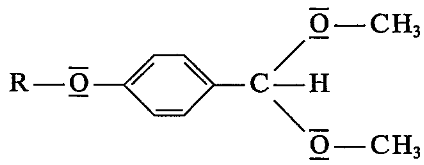

(I)

in der R eine Alkyl-, eine gegebenenfalls substituierte Aryl- oder eine gegebenenfalls substituierte Aralkylgruppe bedeutet, mit einem Katalysatorsystem aus Kobaltcarbonyl und mindestens einem Stickstoff enthaltenden Heterocyclus hydrogenolysiert. Als Edukte in diesem Verfahren kommen insbesondere solche in Betracht, bei denen die Alkylgruppe 1 bis 4 C-Atome enthält und die Arylgruppe Phenyl bedeutet; als Beispiele werden genannt:

4-Methoxy-benzaldehyd-dimethylacetal (Ia),
4-Ethoxy-benzaldehyd-dimethylacetal (Ib),
4-Phenoxy-benzaldehyd-dimethylacetal (Ic),
4-Benzyloxy-benzaldehyd-dimethylacetal (Id) und
4,4′-Diphenylether-dialdehyd-bis-dimethylacetal (Ie = VI).

Die entsprechenden Hauptprodukte der Reaktion sind dann:

(4-Methoxy-benzyl)-methylether (VIIIa),
(4-Ethoxy-benzyl)-methylether (VIIIb),
(4-Phenoxy-benzyl)-methylether (VIIIc),
(4-Benzyloxy-benzyl)-methylether (VIIId) und
4,4′-Bis(methoxymethyl)-diphenylether (VIIIe = VII).

Die Reaktion für diese Beispiele kann durch folgende allgemeine Formel wiedergegeben werden:

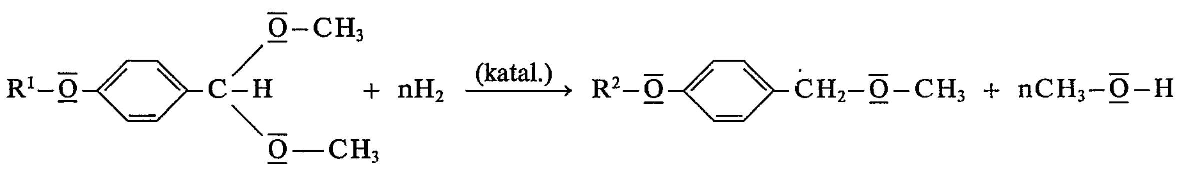


(Ia–e) (VIIIa–e)

| Edukt | $R^1$ | $R^2$ | n | Produkt |
|---|---|---|---|---|
| Ia | $CH_3$ | $CH_3$ | 1 | VIIIa |
| b | $CH_2—CH_3$ | $CH_2—CH_3$ | 1 | b |
| c | 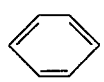 | 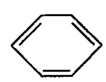 | 1 | c |
| d | 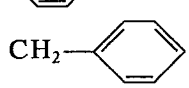 | 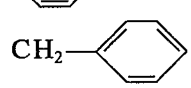 | 1 | d |
| e | 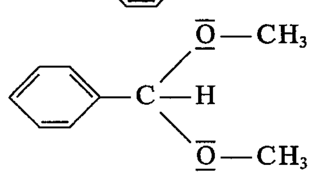 | 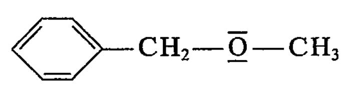 | 2 | e |

Die entstehenden Hauptprodukte im erfindungsgemäßen Verfahren weisen entweder den unveränderten Rest R des Edukts auf (in der allgemeinen Gleichung und der Tabelle ist dann $R^1 = R^2$) oder — sofern der Rest R selbst eine hydrogenolysierende Gruppe wie eine Dimethylacetalgruppe trägt — sie weisen einen gegenüber R veränderten Rest R′ auf (in der allgemeinen Gleichung und der Tabelle ist dann $R^1 \# R^2$).

Von den aufgeführten Verbindungen sind die Edukte (Ia), (Ic) und (Ie) besonders bevorzugt.

Zu den im erfindungsgemäßen Verfahren einzusetzenden Stickstoff enthaltenden Heterocyclen zählen insbesondere Pyridin, Pyrrol, Pyrrolidon und Piperidin, wobei sich Pyridin als besonders geeignet herausgestellt hat; diese Heterocyclen können einzeln oder als Gemisch eingesetzt werden. Durch Zusatz dieser Heterocyclen im erfindungsgemäßen Verfahren zusätzlich zum bekannten Katalysator Kobaltcarbonyl — wobei man im allgemeinen davon ausgeht, daß dieser im Reaktionssystem u. a. in Form von $Co_2(CO)_8$, $Co(CO)_4$ und/oder $HCo(CO)_4$ vorliegen kann — wird das Katalysatorsystem in seiner Selektivität bei der Umwandlung der Aldehyd-acetalgruppe(n) zu(r) Methoxymethyl-Gruppe(n) entscheidend verbessert; d. h., es wird bei der Hydrogenolyse sowohl die Benzyletherspaltung als auch die Einschiebungsreaktion von Kohlenmonoxid unterdrückt. Diese Selektivitätssteuerung ist außerordentlich überraschend, da — wie in der Beschreibung des Standes der Technik dargelegt — der bekannte Zusatz von Phosphorverbindungen hier vollständig versagt und auch die bekannte »Basenreaktion« eher von einem Zusatz der Stickstoff enthaltenden Heterocyclen abgehalten hätte. Im erfindungsgemäßen Verfahren erfolgt jedoch durch den Zusatz der Heterocyclen keine Disproportionierung des Kobaltcarbonyls.

Die Stickstoff enthaltenden Heterocyclen verbessern die Selektivität des Kobaltcarbonyl-Katalysators bereits bei einem molaren Verhältnis von Heterocyclus zu Kobalt (bezogen auf den Kobaltanteil im Kobaltcarbonyl) von etwa 1 zu 1, so daß sie praktisch als Cokatalysatoren angesehen werden können. Der Anteil der Heterocyclen kann bis auf etwa 16 zu 1 gesteigert werden, bevorzugt wird ein Bereich von 2 zu 1 bis 4 zu 1.

Das erfindungsgemäße Verfahren wird zweckmäßig in Methanol als organischem Lösemittel ausgeführt, aber auch andere organische Lösemittel wie Tetrahydrofuran (THF) sind geeignet. Da bei der Durchführung der Reaktion Methanol entsteht, ist dieses als organisches Lösemittel besonders geeignet, zumal es dann durch einfache Destillation wieder in neuen Ansätzen Verwendung finden kann. Der Anteil des zu hydrogenolysierenden Acetals im System ist eher von untergeordneter Bedeutung, er sollte zweckmäßig im Bereich von etwa 10 bis 80 Gew.-% liegen, 40- bis 60gew.-%ige Lösungen sind jedoch bevorzugt, da sie besonders gut handhabbar sind.

Wichtig für einen schnellen und selektiven Ablauf ist die Konzentration des Kobaltcarbonyl-Katalysators (berechnet als Co-Anteil im einkernigen Komplex), bezogen auf die zu hydrogenolysierende Dimethylacetalgruppe; sie liegt zwischen 0,5 und 5 Mol-%, insbesondere zwischen 0,75 und 2 Mol-%, pro Mol Dimethylacetalgruppe. Werden weniger als 0,5 Mol-% an Kobaltcarbonyl-Katalysator eingesetzt, so verläuft die Reaktion zu langsam; bei einer Konzentration von mehr als 5 Mol-% wird bereits die Carbonylierung beispielsweise des bevorzugten organischen Lösemittels Methanol in meßbarem Umfang katalysiert. Das Katalysatorsystem besteht im wesentlichen aus den drei Komponenten a) Kobaltcarbonyl, b) Stickstoff enthaltender Base und c) dem Kohlenmonoxid; ein zweckmäßiges Zusammenwirken dieser Komponenten findet bei folgenden Anteilen statt: 0,5 bis 5 Mol-%, insbesondere 0,75 bis 2 Mol-%, an a), 0,5 bis 20 Mol-%, insbesondere 1,5 bis 8 Mol-%, an b), jeweils bezogen auf 1 Mol Dimethylacetalgruppe, und 20 bis 60 bar, insbesondere 30 bis 50 bar, Partialdruck an Kohlenmonoxid. Das Kohlenmonoxid ist notwendig, um das homogene Katalysatorsystem bei der verhältnismäßig hohen Reaktionstemperatur von etwa 115° bis 150° C, insbesondere 125° bis 135° C, zu stabilisieren. Höhere Kohlenmonoxid-Partialdrücke sind wenig zweckmäßig, weil dann auch in einer Nebenreaktion zunehmend Kohlenmonoxid in die Dimethylacetalgruppe eingeschoben wird (Homologisierung). Zur eigentlichen Hydrogenolyse der Edukte wird nur Wasserstoff verbraucht, so daß im Verlauf bei sinkendem Druck stets nur Wasserstoff zugegeben (nachgedrückt) werden muß. Der Wasserstoff-Partialdruck im erfindungsgemäßen Verfahren kann in einem großen Bereich variiert werden; als untere Grenze sind 20 bar anzusehen, während als zweckmäßige Obergrenze 200 bar gelten können. Bevorzugt wird bei einem Wasserstoff-Partialdruck von 80 bis 120 bar gearbeitet, so daß es ermöglicht wird, die Hydrogenolyse bei einem Gesamtdruck des Systems von weniger als etwa 200 bar durchzuführen. Diese Partialdruck- bzw. Druckangaben sind auf eine Temperatur von etwa 20° C bezogen, sie erhöhen sich bei Temperatursteigerung.

Im folgenden werden die Rahmenbedingungen des erfindungsgemäßen Verfahrens weiter beispielhaft ausgeführt. Der erste Schritt des Verfahrens besteht im Einfüllen oder Herstellen einer Lösung von Kobaltcarbonyl im Druckreaktor. Bei kleineren Ansätzen kann es durchaus zweckmäßig sein, eine Lösung von reinem, festem Kobaltcarbonyl in Methanol oder einem anderen organischen Lösemittel herzustellen und diese dann in der Reaktion einzusetzen. Bei größeren Ansätzen verfährt man jedoch besser so, daß die Lösung des Kobaltcarbonyls aus einer Kobalt in zweiwertiger Form enthaltenden Verbindung im organischen Lösemittel unter Kohlenmonoxid/Wasserstoff-Zugabe unter Druck bei erhöhter Temperatur entweder im Hydrierreaktor selbst oder auch in einem separaten Druckreaktor erzeugt wird; für diese Verfahrensweise geeignete Kobaltverbindungen sind u. a. Co-formiat, -acetat oder -2-ethylhexanoat, die beispielsweise in methanolischer Lösung unter einem Partialdruck von 50 bis 200 bar Kohlenmonoxid, dem zur besseren Reduktion wechselnde Mengen an Wasserstoff zugemischt werden können, bei Temperaturen von 100 bis 140° C leicht zu Kobaltcarbonyl reagieren. Zu der Lösung des Kobaltcarbonyls kann nun die Lösung des Cokatalysators zugegeben werden; es ist jedoch auch möglich, den Stickstoff enthaltenden Heterocyclus erst zusammen mit dem zu hydrogenolysierenden Dimethylacetal ins Reaktionsgemisch einzubringen.

Das Zuführen des Dimethylacetals zur Katalysatorlösung wird in der Regel bei Zimmertemperatur

und drucklos durchgeführt, dies insbesondere, wenn das erfindungsgemäße Verfahren diskontinuierlich ausgeführt wird. Bei kontinuierlicher Reaktionsführung muß jedoch das Dimethylacetal gegen den erhöhten Reaktionsdruck über eine Dosierpumpe in das im Reaktor befindliche Reaktionsgemisch eingepumpt werden. Die eigentliche Reaktionszeit hängt sehr stark von den weiter oben angegebenen übrigen Reaktionsbedingungen ab; um eine effektive Umsetzung der eingesetzten Dimethylacetale zu realisieren, sind beispielsweise Reaktionszeiten von etwa 0,3 bis 5 h zweckmäßig.

Nach abgeschlossener Hydrogenolyse wird das überschüssige Gasgemisch entspannt und die klare, gelbe Lösung aus dem Druckreaktor entnommen und in ein zur Zerstörung des Kobaltcarbonyls geeignetes Reaktionsgefäß, z. B. eine Glaskugel mit Rührer, überführt. Die Zerstörung des Kobaltcarbonyls wird durch Zugabe einer ausreichenden Menge einer Säure unter gleichzeitigem Einleiten von Luft bewirkt, wobei es außerdem zweckmäßig sein kann, dieses Reaktionsgemisch auf etwa 60°C zu erwärmen. Als Säuren zur Ausfällung des oxidativ gebildeten $Co^{2+}$-Ions in der entsprechenden Salzform sind insbesondere Ameisensäure, Oxalsäure oder Phosphorsäure geeignet. Das ausgefallene $Co^{2+}$-Salz wird abfiltriert, und das Filtrat enthält im wesentlichen eine Lösung des gebildeten p-substituierten Benzylmethylethers im organischen Lösemittel, die allerdings durch Nebenprodukte noch leicht verunreinigt ist; die Arten und Anteile der Nebenprodukte werden in den nachfolgenden Beispielen im einzelnen noch ausgeführt. Da im Reaktionsgemisch sowohl Nebenprodukte mit relativ niedrigerem Siedepunkt als der des Hauptproduktes als auch höhermolekulare, nicht-destillierbare Anteile vorliegen können, umfaßt dessen Aufarbeitung noch folgende Schritte: a) eine destillative Abtrennung des organischen Lösemittels, das zweckmäßig in neuen Reaktionsansätzen wiederverwendet wird, b) eine Dünnschichtdestillation des Rückstandes von a) bei vermindertem Druck von etwa 0,1 bis 7 mbar zur Abtrennung der destillativen Produkte von höhermolekularen Produkten, und c) eine alkalische Reinigung des unter b) erhaltenen Destillats, wobei dieses heiß mit z. B. einer 3- bis 20gew.-%igen Natronlauge extrahiert wird, um die Nebenprodukte mit sauren Gruppierungen wie Carbonsäuremethylester aus dem Rohprodukt (Destillat) zu entfernen. Das derart aufbereitete Rohprodukt enthält dann zu mehr als etwa 95 Gew.-% nur noch definierte Verbindungen, deren überwiegender Hauptanteil aus dem gewünschten p-substituierten Benzylmethylether besteht, der in einer Kolonnendestillation im Vakuum in hoher Reinheit von mehr als 99 Gew.-% aus diesem Rohprodukt isoliert werden kann.

Durch das erfindungsgemäße Verfahren von hohem Veredelungsgrad wird beispielsweise ein neuer Weg zur Herstellung von Diethern bzw. Triethern mit aromatischem Mittelteil eröffnet, z. B. zu der industriell wichtigen Verbindung (VII) = (VIIIe), dem 4,4'-Bis-methoxymethyl-di-phenylether. Diese Verbindung ist insbesondere von Wichtigkeit bei der Herstellung aromatischer Polyether aus Hydroxymethyl- oder Alkoxymethyl-diarylethern (siehe z. B. DE-PS 1 252 903 oder US-PS 3 316 186), wobei diese aromatischen Polyether sich als Bindemittel bei der Herstellung von Gußmassen oder als Klebstoff bei der Gewinnung von Schichtkörpern eignen; eine andere bekannte Anwendung dieser Verbindung ist ihre in der DE-OS 2 065 732 (= US-PS 3 867 147) beschriebene Umsetzung mit aromatischen Diazoniumverbindungen zu lichtempfindlichen Kondensationsprodukten aus diesen beiden Komponenten.

In den folgenden Beispielen sind — wenn nichts anderes angegeben ist — unter %-Angaben solche zu verstehen, die auf das Gewicht bezogen sind; Gew.-Teile verhalten sich zu Vol.-Teilen wie g zu $cm^3$.

## Beispiel 1

Die Reaktion wird in einem Druckreaktor aus »V4A«-Stahl ausgeführt, der maximal 200 Vol.-Teile aufnehmen kann; er kann thermostatisiert werden, die Durchmischung des Reaktionsgemisches erfolgt über einen Magnetrührer. Durch eine Deckelbohrung, die während der Druckreaktion verschlossen wird, ist es möglich, im entspannten Zustand flüssige Stoffe zum Reaktionsgemisch zuzugeben bzw. Proben aus diesem zu entnehmen, um so den Verlauf der Umsetzung kontrollieren und steuern zu können.

Das Beispiel wird mit der Bildung von Kobaltcarbonyl gestartet. In den Reaktor wird eine Lösung von 1,5 Gew.-Teilen Kobalt-2-ethyl-hexanoat (16% Co-Gehalt, entsprechend 0,24 Gew.-Teilen bzw. 0,004 Gew.-Teil-Atom Co) in 50 Vol.-Teilen Methanol eingefüllt. Unter einem Druck von 200 bis 240 bar an einem $CO/H_2$-Gemisch im Verhältnis von 1 zu 1 wird auf 130°C erwärmt, wobei die Carbonylbildung nach einer gewissen Induktionsperiode schnell und quantitativ verläuft. Der Druckreaktor wird dann entspannt, und das Edukt wird zusammen mit dem Cokatalysator eingegeben; es werden 36,4 Gew.-Teile 4-Methoxybenzaldehyd-dimethylacetal, verdünnt mit 30 Vol.-Teilen Methanol, und 0,66 Vol.-Teile (= 0,63 Gew.-Teile) Pyridin eingesetzt; die Molanteile der Komponenten betragen dann 2 Mol-% Kobaltcarbonyl und 4 Mol-% Pyridin pro Mol Dimethylacetalgruppe. Anschließend wird erneut ein $CO/H_2$-Gemisch aufgedrückt. Der Partialdruck des Kohlenmonoxids beträgt 40 bar, der Partialdruck des Wasserstoffes etwa 80 bar; beide Werte werden bei ca. 20°C ermittelt, die Reaktion selbst verläuft bei etwa 130°C, wobei der Druck im Reaktor auf maximal etwa 160 bar ansteigt. Der Reaktionsverlauf ist deutlich am Druckabfall (Wasserstoffabnahme) zu erkennen, nach etwa 20 min Reaktionszeit wird die Umsetzung durch schnelles Abkühlen gestoppt. Nach völliger Abkühlung auf

Raumtemperatur wird das noch überschüssig vorhandene Gasgemisch entspannt und die Reaktionslösung dem Druckreaktor entnommen.

Zur Zerstörung des Kobaltcarbonyls werden 1,46 Gew.-Teile Ameisensäure zur Reaktionslösung zugegeben, und diese wird unter Durchleiten von Luft auf etwa 60°C erwärmt. Das entstehende Co-formiat kristallisiert zu etwa 70 bis 80% aus und kann leicht abfiltriert werden. Das erhaltene Filtrat ist frei von Kobaltcarbonyl, und aus ihm wird am Rotationsverdampfer das organische Lösemittel Methanol entfernt. Das zurückbleibende Rohprodukt wird durch Gaschromatographie (Trägergas: He, Standard: Phenoxytoluol) analysiert. Die erhaltenen Anteile sind auf den 100%igen Umsatz bezogen und der nachfolgenden Tabelle zu entnehmen.

Beispiele 2 und 3 und Vergleichsbeispiele V 1 und V 2

Es wird nach den Angaben des Beispiels 1 verfahren, aber mit wechselnden Mengen an Cokatalysator Pyridin bzw. ohne diesen (siehe Tabelle). Die Ergebnisse der erfindungsgemäßen und der Vergleichsbeispiele zeigen signifikant den Einfluß des Cokatalysators auf den relativen Ertrag an p-substituiertem Benzylmethylether.

| Beispiel | 1 | 2 | 3 | V 1 | V 2 |
|---|---|---|---|---|---|
| Mol.-% an Kobaltcarbonyl | 2 | 2 | 2 | 2 | 2 |
| Mol.-% am Pyridin | 4 | 2 | 8 | – | – |
| Partialdruck des CO in bar | 40 | 40 | 40 | 40 | 40 |
| Partialdruck des $H_2$ in bar | 80 | 80 | 80 | 80 | 80 |
| Reaktionstemperatur in °C | 130 | 130 | 130 | 130 | 130 |
| Reaktionszeit in min | 20 | 20 | 30 | 20 | 15 |
| Umsatz des Edukts in % | 100 | 100 | 88 | 100 | 92 |
| Selektivität in % an (hochgerechnet auf 100%igen Umsatz des Edukts) | | | | | |
| 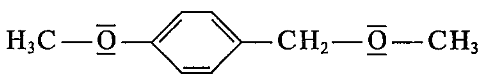 4-Methoxybenzyl-methylether | 84 | 68 | 97 | 10 | 12 |
| 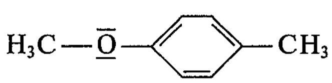 4-Methoxytoluol | 6 | 6 | 1 | 49 | 49 |
| 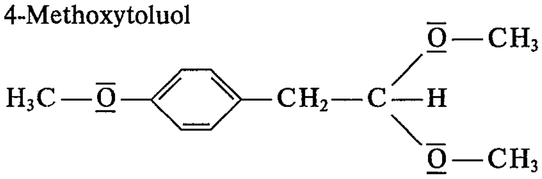 (4-Methoxyphenyl)-acetaldehyddimethylacetal | 10 | 26 | 1 | 40 | 38 |

Beispiel 4 und Vergleichsbeispiel V 3

Es wird nach den Angaben des Beispiels 1 verfahren, aber es werden als Edukt 32 Gew.-Teile 4,4′-Diphenylether-dialdehyd-bis-dimethylacetal eingesetzt; die Molanteile der Komponenten betragen damit ebenfalls 2 Mol-% Kobaltcarbonyl und 4 Mol-% Pyridin pro Mol Dimethylacetalgruppe. Die Reaktion selbst wird bei etwa 130° bis 135°C durchgeführt, die Reaktionszeit beträgt 150 min. Im Vergleichsbeispiel V 3 wird unter sonst gleichen Bedingungen das Pyridin weggelassen. Bei dem 100%igen Umsatz an Edukt werden folgende Produkte in den nachstehenden Selektivitäten (durch GC bestimmt) erhalten:

| Produkt | Selektivität im Beispiel 4 | V 3 |
|---|---|---|
| 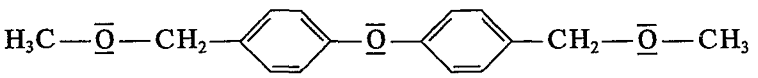<br>4,4'-Bis(methoxymethyl)-diphenylether | 93,0% | 26,0% |
| 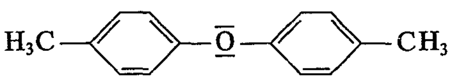<br>4,4'-Dimethyl-diphenylether | 0,5% | 7,1% |
| 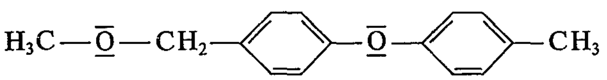<br>4-Methoxymethyl-4'-methyl-diphenylether | 3,5% | 15,7% |
| 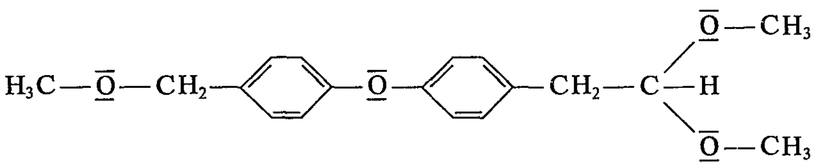<br>4-Methoxymethyl-4'-dimethoxyethyl-diphenylether | 2,3% | 19,7% |

Im Vergleichsbeispiel V 3 werden noch eine Reihe weiterer Nebenprodukte erhalten, die im erfindungsgemäßen Beispiel 4 nicht oder nur in Spuren nachzuweisen sind.

Beispiel 5

Die Bedeutung des Zusatzes von Pyridin zur Selektivitätssteigerung zeigt sich besonders deutlich, wenn als Edukt nicht durch Destillation gereinigtes, sondern rohes 4,4'-Diphenylether-dialdehyd-bis-dimethylacetal eingesetzt wird; in der Rohform fällt dieses Edukt nach der elektrochemischen Methoxylierung des 4,4'-Dimethyl-diphenylethers an (siehe einleitenden Teil der Beschreibung zum Stand der Technik), wobei eine Zwischenreinigung in großtechnischen Verfahren oftmals von Nachteil sein kann, da sie zumindest aufwendig ist.

Der Druckreaktor kann maximal 2000 Vol.-Teile aufnehmen, es werden 15 Gew.-Teile Kobalt-2-ethyl-hexanoat (entsprechend 2,4 Gew.-Teilen bzw. 0,04 Gew.-Teil-Atom Co) in 500 Vol.-Teilen Methanol eingesetzt; das $CO/H_2$-Gemisch wird bis zu einem Druck von etwa 200 bar eingegeben und der Ansatz auf etwa 135°C erwärmt. Als Edukt werden 640 Gew.-Teile des rohen Bis-dimethylacetals in 300 Vol.-Teilen Methanol und 6,34 Gew.-Teile Pyridin zugegeben. Ein Gasgemisch mit einem CO-Partialdruck von 40 bar und einem $H_2$-Partialdruck von 80 bar wird dem Reaktionsgemisch aufgedrückt, der Druck erreicht bei einer Erwärmung auf etwa 130°C einen Wert von etwa 155 bar; etwa 40 min nach Reaktionsbeginn ist der Druck auf ca. 100 bar abgesunken, und es wird $H_2$ bis zum Gesamtdruck von 160 bar nachgedrückt. Nach weiteren 180 min ist der Druck auf etwa 120 bar abgesunken und die Reaktion beendet. Zur Zerstörung des Kobaltcarbonyls werden 14,6 Gew.-Teile Ameisensäure zum abgekühlten Reaktionsgemisch gegeben. Nach Abtrennen des organischen Lösemittels Methanol werden 580 Gew.-Teile eines Rohproduktes erhalten, das einer Dünnschichtdestillation bei 0,3 mbar und 190°C Heizmanteltemperatur unterworfen wird, woraus 503 Gew.-Teile Destillat und 70 Gew.-Teile undestillierbarer Rückstand resultieren. Die sich anschließende Destillation über eine Kolonne wird erleichtert, wenn das Dünnschichtdestillat noch mit etwa dem gleichen Volumen an 10%iger wäßriger NaOH-Lösung bei 100°C extrahiert wird. Durch diese Zwischenreinigung werden Carbonsäuren, deren Methylester sowie andere Nebenprodukte sauren Charakters abgetrennt. Die anschließende fraktionierte Destillation liefert einen Vorlauf bei etwa 84° bis 204°C (Vakuum von 6,5 mbar) von 52 Gew.-Teilen und einen Hauptlauf bei 204°C von 382 Gew.-Teilen, die sich wie folgt zusammensetzen:

Vorlauf:
7 Gew.-Teile 4,4'-Dimethyl-diphenylether (=2%)
31 Gew.-Teile 4-Methoxymethyl-4'-methyl-diphenylether (=8%)
14 Gew.-Teile 4,4'-Bis(methoxymethyl)-diphenylether (=3%)

Hauptlauf:

382 Gew.-Teile 4,4'-Bis(methoxymethyl)-diphenylether (=85%)

Es verbleibt nur ein sehr geringer Destillationsrückstand, das gewünschte Hauptprodukt ist farblos und verfärbt sich auch nicht bei längerem Stehen.

Vergleichsbeispiel V 4

Es wird nach den Angaben des Beispiels 5 verfahren, aber ohne den Zusatz des Cokatalysators Pyridin. Die Reaktion wird bei gleichem Gasdruck, aber bei 125°C (statt 130°C im Beispiel 5) ausgeführt; die Reaktion ist bereits nach 90 min beendet, d. h., nach dieser Zeit ist das Edukt vollständig umgesetzt. Nach Abtrennen des organischen Lösemittels verbleiben 550 Gew.-Teile eines Rohproduktes, dessen GC-Analyse 21% an 4,4'-Bis(methoxymethyl)-diphenylether nachweist. Die Dünnschichtdestillation dieses Rohproduktes liefert 440 Gew.-Teile Destillat und einen erhöhten Anteil von 105 Gew.-Teilen nicht-destillierbarem Rückstand. Die nachfolgende NaOH-Extraktion verläuft verschieden von der des Beispiels 5, da sich feste Produkte bilden, die sich störend an der Flüssig-Flüssig-Phasengrenze ansammeln und die Phasentrennung praktisch unmöglich machen; sie gelingt erst nach Zugabe eines Hilfslösemittels wie Toluol, das aber wieder aufwendig destillativ entfernt werden muß. Die fraktionierte Destillation des so zwischengereinigten Produktes liefert einen ersten Bereich bei etwa 59° bis 179°C (Vakuum von 2,6 mbar) von 132 Gew.-Teilen, einen zweiten Bereich bei etwa 162° bis 164°C (Vakuum von 1,3 mbar) von 90 Gew.-Teilen und einen letzten Bereich bei etwa 165° bis 192°C (Vakuum von 1,3 mbar) von 125 Gew.-Teilen neben einigen harzartigen Rückständen, wobei folgende Zusammensetzung auftritt:

1. Bereich:

26 Gew.-Teile 4,4'-Dimethyl-diphenylether (8%)
91 Gew.-Teile 4-Methoxymethyl-4'-methyl-diphenylether (25%)
12 Gew.-Teile 4,4'-Bis(methoxymethyl)-diphenylether (2%)

2. Bereich:

87 Gew.-Teile 4,4-Bis(methoxymethyl)-diphenylether (19%)

3. Bereich:

24 Gew.-Teile 4(4'-Methoxymethyl-phenoxy)-benzoesäure-methylester (5%)
73 Gew.-Teile 4-Methoxymethyl-4'-dimethoxyethyl-diphenylether (15%)

und weitere Komponenten unbekannter Zusammensetzung. Der isolierte 4,4'-Bis(methoxymethyl)-diphenylether beträgt also nur etwa 19% d. Th.; er enthält außerdem noch Verunreinigungen, die zur Gelbfärbung auch nach der Destillation führen.

Beispiel 6

Es wird nach den Angaben des Beispiels 1 verfahren, aber mit 0,75 Gew.-Teilen Kobalt-2-ethyl-hexanoat (entsprechend 0,002 Gew.-Teil-Atom Co), 300 bar an $H_2$/CO-Gemisch in der Herstellstufe des Kobaltcarbonyls, 25 Gew.-Teilen 4-Phenoxybenzaldehyd-dimethylacetal und 0,33 Vol.-Teilen Pyridin; die Molanteile der Komponenten betragen dann 2 Mol-% Kobaltcarbonyl und 4 Mol-% Pyridin pro Mol Dimethylacetalgruppe. Nach etwa 60 min Reaktionszeit der Hydrogenolyse werden 20 bar $H_2$ nachgedrückt, die Gesamtreaktionszeit beträgt etwa 120 min, wobei der Umsatz an Edukt 100% ausmacht. Das erhaltene Rohprodukt setzt sich (nach GC-Analyse bzw. destillativer Aufarbeitung) zusammen aus:

92% 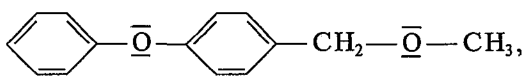, (4-Phenoxybenzyl)-methylether

1% 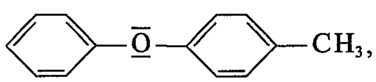, 4-Phenoxytoluol

4% weiteren Nebenprodukten, und

3% nicht-destillierbaren Anteilen.

**Patentansprüche**

1. Verfahren zur katalytischen Hydrogenolyse von p-substituierten Benzaldehyd-dialkylacetalen mit einem Kobaltcarbonyl-Katalysator und einem Gemisch aus Wasserstoff/Kohlenmonoxid zu den entsprechenden p-substituierten Benzylalkylether-Derivaten, dadurch gekennzeichnet, daß man Verbindungen der allgemeinen Formel (I)

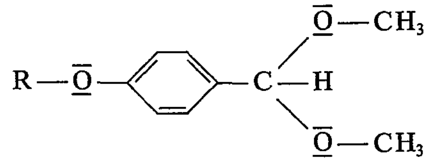

(I)

in der R eine Alkyl-, eine gegebenenfalls substituierte Aryl- oder eine gegebenenfalls substituierte Aralkylgruppe bedeutet, mit einem Katalysatorsystem aus Kobaltcarbonyl und mindestens einem Stickstoff enthaltenden Heterocyclus hydrogenolysiert.

2. Verfahren nach Anspruch 1, dadurch gekennzeichnet, daß die Alkylgruppe 1 bis 4 C-Atome enthält und die Arylgruppe Phenyl bedeutet.

3. Verfahren nach Anspruch 1 oder 2, dadurch gekennzeichnet, daß man 4-Methoxy-benzaldehyd-dimethylacetal, 4-Phenoxy-benzaldehyd-dimethylacetal oder 4,4'-Diphenylether-dialdehyd-bis-dimethylacetal hydrogenolysiert.

4. Verfahren nach einem der Ansprüche 1 bis 3, dadurch gekennzeichnet, daß ein Stickstoff enthaltender Heterocyclus aus der Gruppe Pyridin, Pyrrol, Pyrrolidon und Piperidin eingesetzt wird.

5. Verfahren nach einem der Ansprüche 1 bis 4, dadurch gekennzeichnet, daß der molare Anteil des Stickstoff enthaltenden Heterocyclus zu Kobaltcarbonyl im Bereich von 1 zu 1 bis 16 zu 1 liegt.

6. Verfahren nach einem der Ansprüche 1 bis 5, dadurch gekennzeichnet, daß die Konzentration an Kobaltcarbonyl zwischen 0,5 und 5 Mol-% pro Mol Dimethylacetalgruppe beträgt.

7. Verfahren nach einem der Ansprüche 1 bis 6, dadurch gekennzeichnet, daß die Anteile an Kobaltcarbonyl 0,5 bis 5 Mol-% und an Stickstoff enthaltender Base 0,5 bis 20 Mol-%, jeweils bezogen auf 1 Mol Dimethylacetalgruppe, und der Partialdruck des Kohlenmonoxids 20 bis 60 bar betragen.

8. Verfahren nach einem der Ansprüche 1 bis 7, dadurch gekennzeichnet, daß die Anteile an Kobaltcarbonyl 0,75 bis 2 Mol-% und an Stickstoff enthaltender Base 1,5 bis 8 Mol-%, jeweils bezogen auf 1 Mol Dimethylacetalgruppe, und der Partialdruck des Kohlenmonoxids 30 bis 50 bar betragen.

**Claims**

1. A process for the catalytic hydrogenolysis of a p-substituted benzaldehyde-dialkylacetal by means of a cobalt carbonyl catalyst and a hydrogen/carbon monoxide mixture to give the corresponding p-substituted benzyl alkyl ether derivative, which comprises hydrogenolyzing a compound of the general formula (I)

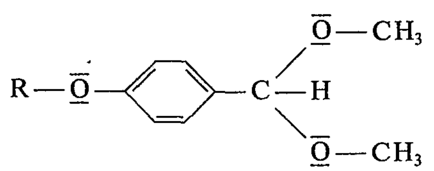

(I)

in which R is an alkyl group, an unsubstituted or substituted aryl group or an unsubstituted or substituted aralkyl group, by means of a catalyst system composed of cobalt carbonyl and at least one nitrogen-containing heterocyclic compound.

2. A process as claimed in claim 1, wherein the alkyl group contains 1 to 4 C atoms and the aryl group is phenyl

3. A process as claimed in claim 1 or 2, wherein 4-methoxy-benzaldehyde-dimethylacetal, 4-phenoxy-benzaldehyde-dimethylacetal or 4,4'-diphenyl ether-dialdehyde-bis-dimethylacetal is hydrogenolyzed.

4. A process as claimed in any of claims 1 to 3, wherein the nitrogen-containing heterocyclic compound used is selected from the group comprising pyridine, pyrrole, pyrrolidone and piperidine

5. A process as claimed in any of claims 1 to 4, wherein the molar ratio of the nitrogen-containing heterocyclic compound to cobald carbonyl is in the range from 1 : 1 to 16 : 1.

6. A process as claimed in any of claims 1 to 5, wherein the concentration of cobalt carbonyl is between 0.5 and 5 mole % per mole of dimethylacetal group.

7. A process as claimed in any of claims 1 to 6, wherein the proportion of cobalt carbonyl is 0.5 to 5 mole % and that of nitrogen-containing base is 0.5 to 20 mole %, each relative to 1 mole of dimethylacetal group, and the carbon monoxide partial pressure is 20 to 60 bars.

8. A process as claimed in any of claims 1 to 7, wherein the proportion of cobalt carbonyl is 0.75 to 2 mole % and that of nitrogen-containing base is 1.5 to 8 mole %, each relative to 1 mole of dimethylacetal group, and the carbon monoxide partial pressure is 30 to 50 bars.

**Revendications**

1. Procédé pour l'hydrogénolyse catalytique de benzaldéhydedialcoylacétals p-substitués avec un catalyseur de cobalt carbonyle et un mélange d'hydrogène/oxyde de carbone, en les dérivés de benzylalcoyléthers p-substitués correspondants, caractérisé en ce qu'on hydrogénolyse des composés de formule générale (I)

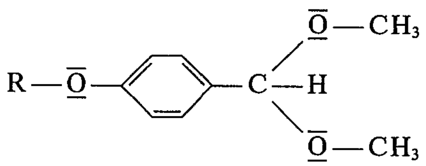

(I)

dans laquelle R représente un groupe alcoyle, un groupe aryle éventuellement substitué ou un groupe aralcoyle éventuellement substitué, avec un système de catalyseur constitué de cobalt carbonyle et d'au moins un composé hétérocyclique contenant de l'azote.

2. Procédé selon la revendication 1, caractérisé en ce que le groupe alcoyle contient de 1 à 4 atomes de carbone et le groupe aryle est le groupe phényle.

3. Procédé selon la revendication 1 ou la revendication 2, caractérisé en ce qu'on hydrogénolyse le 4-méthoxy-benzaldéhyde-diméthalacétal, le 4-phénoxy-benzaldéhyde-diméthylacétal ou le 4,4'-diphénylétherdialdéhyde-bis-diméthylacétal.

4. Procédé selon l'une des revendications 1 à 3, caractérisé en ce qu'on utilise un composé hétérocyclique contenant de l'azote, choisi parmi la pyridine, le pyrrole, la pyrrolidone et la pipéridine.

5. Procédé selon l'une des revendications 1 à 4, caractérisé en ce que la proportion molaire du composé hétérocyclique contenant de l'azote au cobalt carbonyle est comprise entre 1 : 1 et 16 : 1.

6. Procédé selon l'une quelconque des revendications 1 à 5, caractérisé en ce que la concentration en cobalt carbonyle est comprise entre 0,5 et 5 moles % par mole de groupe diméthylacétal.

7. Procédé selon l'une quelconque des revendications 1 à 6, caractérisé en ce que les proportions sont pour le cobalt carbonyle de 0,5 à 5 moles % et pour la base contenant de l'azote de 0,5 à 20 moles %, par mole de groupe diméthylacétal, et la pression partielle de l'oxyde de carbone atteint de 20 à 60 bars.

8. Procédé selon l'une quelconque des revendications 1 à 7, caractérisé en ce que les proportions sont pour le cobalt carbonyle de 0,75 à 2 moles %, et pour la base contenant de l'azote de 1,5 à 8 moles %, par mole de groupe diméthylacétal, et la pression partielle d'oxyde de carbone atteint de 30 à 40 bars.